Europäisches Patentamt

European Patent Office

Office européen des brevets

(19)

(11) Veröffentlichungsnummer : **0 021 190**
**B1**

(12)

# EUROPÄISCHE PATENTSCHRIFT

(45) Veröffentlichungstag der Patentschrift :
11.05.83

(21) Anmeldenummer : 80103157.6

(22) Anmeldetag : 06.06.80

(51) Int. Cl.³ : **G 08 B 21/00,** G 01 N 33/00

(54) **Vorrichtung zur individuellen Überwachung der Exposition einer Person gegenüber toxischen Gasen.**

(30) Priorität : 16.06.79 DE 2924367

(43) Veröffentlichungstag der Anmeldung :
07.01.81 Patentblatt 81/01

(45) Bekanntmachung des Hinweises auf die Patenterteilung : 11.05.83 Patentblatt 83/19

(84) Benannte Vertragsstaaten :
**CH DE FR GB IT LI NL SE**

(56) Entgegenhaltungen :
DE A 2 713 623
FR A 2 382 690
FR A 2 408 834
US A 4 073 621

(73) Patentinhaber : **BAYER AG**
**Zentralbereich Patente, Marken und Lizenzen**
**D-5090 Leverkusen 1, Bayerwerk (DE)**

**Compur-Electronic GmbH**
**Steinerstrasse 15**
**D-8000 München 70 (DE)** ·

(72) Erfinder : **Drope, Eckard, Dr.**
**Wiener Weg 58**
**D-5000 Köln 40 (DE)**
Erfinder : **Jansky, Karl Peter**
**Hauserweg 4a**
**D-8193 Muensing (DE)**
Erfinder : **Pross, Wilhelm**
**Oberstdorfer Strasse 8**
**D-8000 München 71 (DE)**
Erfinder : **Breuer, Wolfram, Dr.**
**Walter-Flex-Strasse 16**
**D-5090 Leverkusen (DE)**

Jouve, 18, rue St-Denis, 75001 Paris, France

EP 0 021 190 B1

# Vorrichtung zur individuellen Überwachung der Exposition einer Person gegenüber toxischen Gasen

Die Erfindung betrifft eine Vorrichtung zur individuellen Überwachung der Exposition einer Person gegenüber toxischen Gasen mit einem Gassensor, der die Gaskonzentration in ein elektrisches Signal umwandelt, einer Einrichtung zur Aufzeichnung des vom Sensor erfaßten Konzentrationsverlaufs und einer Schaltung zur Bildung von Konzentrationsmittelwerten, sowie einem bei der Überschreitung eines kritischen Mittelwertes ansprechenden Alarmgeber.

In vielen Industriebetrieben sind die Mitarbeiter den unterschiedlichsten Belastungen durch toxische Gase ausgesetzt. Die Exposition einer Person gegenüber einem toxischen Gas ist physikalisch definiert als das Integral der jeweils am Ort der Person gemessenen Gaskonzentration über die Zeit. Von besonderem Interesse ist die während der Arbeits- oder Schichtzeit der betreffenden Person kumulierte Gesamtexposition bzw. ein daraus abgeleiteter zeitlicher Mittelwert. Gasdetektoren für den Arbeitsschutz, die am Körper des Mitarbeiters getragen werden, sind sowohl im Aufbau wie in ihren Leistungen sehr unterschiedlich, wobei für die gewählte Aussage vorrangig das Meßprinzip entscheidend ist. So existieren Detektoren, die einen Alarm auslösen, wenn eine Überschreitung der maximal zulässigen Arbeitsplatzkonzentration eines toxischen Gases erkannt wird. Diese Detektoren benutzen gewöhnlich elektrochemische Sensoren, die sich durch geringe Ansprechzeiten auszeichnen (DE-PS-2 621 676). Weiterhin sind integrierende Geräte bekannt, die den Schichtmittelwert ermitteln. Diese Geräte basieren auf einem Luftprobensammler, bei dem mit Hilfe einer Kleinpumpe die am Arbeitsplatz vorherrschende Luft kontinuierlich durch einen Absorber gesaugt wird. Im Absorber wird der zu registrierende Schadstoff festgehalten (DE-OS-26 58 739).

In der DE-OS-2 713 623 wird ein Gerät zur Messung einer Gasdosis beschrieben, wobei die Möglichkeit Dosisalarm zu geben, vorgesehen ist. Das Gerät beruht darauf, daß aus der zu untersuchenden Atmosphäre kontinuierlich ein Probenstrom entnommen und in ein Gefäß mit veränderlichem Volumen unter konstantem Druck eingeblasen wird und die in dem Gefäß vorhandene Gaskonzentration kontinuierlich bestimmt wird. Abgesehen davon, daß die Bedingung der Druckkonstanz im Mischgefäß in der Praxis kaum zu realisieren ist, sind auch die Voraussetzungen für die Ausführung des Gerätes als tragbares und personenbezogenes Meßgerät nicht gegeben. Außerdem ist es — bedingt durch das Meßprinzip — nicht möglich, die Gasbelastung in aufeinanderfolgenden Zeitabschnitten (sequentielle Dosismessung) zu messen. Durch die Gassammeleinrichtung erfolgt eine fortlaufende Aufsummierung (Integration) des Meßgases. Das Gerät kann also nur die jeweils bis zu einem bestimmten Zeitpunkt kumulierte Gesamtdosis messen. Wird die maximal zulässige Dosis erreicht, so muß das Gerät abgeschaltet und die Gassammeleinrichtung gespült und wieder neu mit reiner Luft gefüllt werden. Erst nach diesen Manipulationen ist das Gerät wieder für eine neue Dosismessung bereit.

Ferner ist in dem US-Patent 4 073 621 ein Gerät beschrieben, das mit Hilfe eines Indikatorpapierstreifens eine mit der Konzentration verwandte Größe in Form der Verfärbung des Papierstreifens aufzeichnet. Mit einem Fotometer kann anschließend der Papierstreifen ausgewertet werden. Der Meßeffekt (Verfärbung) wird hervorgerufen durch die Exposition, d. h. durch die Einwirkung des Gases während einer ganz bestimmten Zeit, die durch die Papiertransportgeschwindigkeit vorgegeben ist, da die Verfärbung proportional zum Produkt aus Gaskonzentration und Einwirkungszeit ist. Die führt dazu, daß schnelle Konzentrationsspitzen, die z. B. während eines Zeitintervalles von 10 bis 30 Sekunden auftreten, nicht erfaßt werden. Ferner wird bei diesem Gerät nur die gesamte Gasdosis während eines Arbeitstages oder einer Arbeitsschicht bestimmt. Die laufende Erfassung und Dokumentation von Kurzzeitmittelwerten ist nicht vorgesehen.

Bei einer neueren Version ist das Fotometer direkt mit in das Gerät integriert und löst einen Alarm aus, wenn der Mittelwert der Gaskonzentration während der durch die Transportgeschwindigkeit des Papierstreifens festgelegten Mittelungszeit einen Grenzwert übersteigt. Es wird also Alarm gegeben, wenn während der durch das Gerät vorgegebenen Mittelungszeit eine bestimmte Gasdosis überschritten wird (Dosiswarnung).

Nach dem Stand der Technik werden also die verschiedenen Meßprobleme, nämlich die Erfassung der Momentanwertkonzentration mit Alarmgabe bei Grenzwertüberschreitung, die Dosiswarnung, die Erfassung des Konzentrationsverlaufs und des Schichtmittelwertes durch unterschiedliche Geräte gelöst.

Für die Überwachung einer Person hinsichtlich der Exposition gegenüber einem toxischen Gas ist aber die Kombination aller dieser Aussagen wichtig, wobei die Richtung und die Mittelungszeiten gasspezifisch festzulegen sind : Liegt bei den akut toxischen Stoffen der Schwerpunkt auf der momentanen Warnung, so kommt es bei Stoffen, deren Wirkung eine mittelgroße Halbwertszeit hat, auf die Einstellung der richtigen Warndosis an. Dagegen ist bei den kumulativ wirkenden Stoffen die Warnfunktion von untergeordneter Bedeutung. Die Erfassung des Konzentrationsverlaufes erfolgt schließlich zu einem gänzlich anderen Zweck, nämlich der Erkennung von Arbeitsgängen, die mit einer besonders starken Gasbelastung verbunden sind. Hierfür ist nur eine geringe zeitliche Auflösung (einige Minuten) erforderlich, da sonst die nichtsystematischen kurzen Schwankungen eine falsche Bewertung ergeben würden. Der Schichtmittelwert (8-Stunden-Mittelwert) läßt sich schließlich definitionsgemäß mit dem MAK-Wert (s. Veröffentlichungen über Arbeitsschutz) vergleichen.

Eine weitere Komplikation entsteht dadurch, daß für unterschiedliche Arbeitsstoffe (Gase) häufig ganz unterschiedliche Meßverfahren herangezogen werden müssen. Ein vollständiges Geräteprogramm für den Arbeitsschutz besteht daher aus den unterschiedlichsten Komponenten.

Der Erfindung liegt nun die Aufgabe zugrunde, eine Vorrichtung zu entwickeln, die die drei Teilprobleme, d. i. Momentanwertalarm, Doiswarnung und Feststellung des Konzentrationsprofils, sowie des Schichtmittelwertes für beliebige Sensoren auf der Basis einer einheitlichen Gerätekonzeption löst.

Die erfindungsgemäße Lösung dieser Aufgabe ist gekennzeichnet durch

a) einen Schalter, der das vom Sensor kommende elektrische Signal periodisch alle 1 bis 10 Sekunden, vorzugsweise 1 bis 2 Sekunden, abfragt, und einen mit dem Sensor verbundenen weiteren Alarmgeber,

b) einen AD-Wandler, der den abgefragten Signalwert digitalisiert,

c) eine erste Integrierstufe, die die digitalisierten Meßwerte für eine Mittelwertbildung über einen vorgegebenen Zeitabschnitt $\tau$ integriert, wobei $\tau$ größer ist als die Zeitkonstante des Sensors und 50 bis 150 Sekunden beträgt,

d) einen Speicher, der die durch die Integrierstufe gemittelten Meßwerte speichert,

e) eine zweite Integrierstufe, die aus den jeweils letzten n Mittelwerten den fließenden Mittelwert über einen Zeitabschnitt $n \cdot \tau$ bildet, wobei $2 \leqslant n \leqslant 40$ ist,

f) einen Komparator, der den fließenden Mittelwert mit einem vorgegebenen Grenzwert vergleicht und mit dem Alarmgeber verbunden ist.

Weiterentwicklungen der Erfindung sind in den Unteransprüchen angegeben.

Der technische Fortschritt der Erfindung liegt in der einheitlichen Problemlösung für den individuellen Arbeitsschutz. Auf der Basis dieser Lösung wurde zum ersten Mal ein am Körper tragbares Dosiswarngerät für toxische Gase geschaffen, das einerseits Alarmfunktionen (Momentanwertalarm und Mittelwertalarm) besitzt und andererseits eine nachträgliche Dokumentation des Expositionsverlaufs (Gaskonzentration als Funktion der Zeit) gestattet. Die dokumentierten Daten können dann von einem externen Rechner, z. B. zur Bestimmung von Langzeitmittelwerten, weiter verarbeitet werden. Die erfindungsgemäße Vorrichtung schafft die Voraussetzungen, daß die oben angegebenen Funktionen auf ·elektronischem Wege in einem einzigen Gerät realisiert werden können. Beim Übergang auf eine andere Gaskomponente braucht lediglich der Sensor ausgewechselt zu werden. Das Gerät bietet ferner den Vorteil, daß es sich entsprechend den gasspezifischen Erfordernissen leicht modifizieren läßt. So wird z. B. bei Gasen akuter Toxizität in jedem Falle Alarm ausgelöst, wenn der Momentanwert einen kritischen Wert überschreitet, während bei kumulativ wirkenden Gasen die Alarmauslösung im Falle der Mittelwertüberschreitung im Vordergrund steht. Von großer praktischer Bedeutung sind schließlich die Möglichkeiten zur Miniaturisierung. Das zugrundeliegende Konzept kann in einem relativ kleinen Gehäuse mit modernen elektronischen Mitteln realisiert werden. Dabei sind zwei Gesichtspunkte maßgebend :

1. Es werden nur Mittelwerte und nicht Momentanwerte gespeichert. Auf diese Weise kommt man mit einer relativ geringen Speicherkapazität aus.

2. Die Abfrage des Speichers erfolgt mit einer externen Auswertezentrale, an die das Gerät angeschlossen werden kann.

In dem Gerät sind also nur die Meßwertabfrage, die Mittelwertbildung, die Speicherung und die Alarmfunktionen integriert. Diese Separierung bildet eine wesentliche Voraussetzung bei der Erfüllung der Forderung nach möglichst geringem Bauvolumen.

Im folgenden wird ein Ausführungsbeispiel der Erfindung anhand eines Blockdiagramms beschrieben. Zur Erfassung der Gaskonzentration dient ein Sensor 1, der ein mit der Konzentration ansteigendes elektrisches Signal erzeugt. Zweckmäßig werden hierfür elektrochemische Sensoren benutzt, wie sie z. B. in der DE-PS 2 621 676 beschrieben sind. Der Meßwert wird von einem Schalter 2 periodisch abgefragt. Der Schaltzyklus beträgt vorzugsweise 1 bis 2 Sekunden. Nur bei sehr langsamen Konzentrationsänderungen können auch kleinere Schaltfrequenzen, d. h. längere Zykluszeiten (bis zu 10 Sekunden), angewandt werden. Anschließend werden die abgefragten Meßwerte in einem A/D-Wandler 3 digitalisiert und mit Hilfe einer Korrekturschaltung 4 eine Linearisierung durchgeführt, wenn der Sensor 1 eine nicht-lineare Charakteristik hat. Die Korrekturschaltung 4 sorgt also für eine Linearisierung der Kennlinie. Die so modifizierten und digitalisierten Meßwerte werden einem Integrator 5 zugeführt, der die Meßwerte für eine Mittelwertbildung über einen vorgegebenen Zeitabschnitt $\tau$ integriert. Der Zeitabschnitt $\tau$ wird dabei so gewählt, daß der systematische Konzentrationsverlauf sichtbar wird, während Momentanwertschwankungen unterdrückt werden. Bei der Wahl von $\tau$ muß ferner die Zeitkonstante des Sensors berücksichtigt werden. $\tau$ darf nicht unter der Größenordnung der Sensorzeitkonstante liegen. In der Praxis werden für die Mittelwertbildung Zeitabschnitte $\tau$ von 50-150 s gewählt. Die gebildeten Mittelwerte werden in den Speicher 6 übernommen. Die gespeicherten Meßwerte werden dann in einem zweiten Integrator 7 erneut gemittelt, wobei die Mittelung über einen wesentlich längeren Zeitabschnitt $n \cdot \tau$ erfolgt (mit $2 \leqslant n \leqslant 40$). Die Festlegung von n richtet sich nach der Toxizität des zu messenden Gases. In Zweifelsfällen müssen bei kritischen Gasen relativ kleine n-Werte zugrundegelegt werden. Der im Integrator 7 vollzogene Schritt soll hier als fließende Mittelwertbildung über den Zeitabschnitt n · τ bezeichnet werden. Mit dem Integrator 7 verbunden ist ein Komparator, der den fließenden Mittelwert mit einem vorgegebenen Grenzwert vergleicht und ein Alarmsignal (Alarmgeber 8) auslöst, wenn der Grenzwert überschritten wird.

Die Komponenten 2 bis 8 sind in einer Baueinheit zusammengefaßt, die als Zusatzgerät zum Gasdetektor 1 geliefert wird. Dieses Zusatzgerät ist seiner Funktion nach ein Dosismesser mit Alarmfunktion 8 und eingebautem Speicher 6. Ein zweiter Alarmgeber (nicht gezeichnet) kann direkt an den Sensor angeschlossen werden und warnt bei Grenzwertüberschreitungen des Momentanwertes der Gaskonzentration.

Die in 6 abgespeicherten Mittelwerte können außerdem mit Hilfe einer externen Auswertezentrale ausgelesen und dokumentiert werden. Die Auswertezentrale besteht hier aus dem D/A-Wandler 9, dem Logarithmierer 10 und dem X-Y-Schreiber 11. Zunächst werden die vom Speicher 6 abgefragten Meßwerte in Analogwerte umgesetzt 9, anschließend logarithmiert 10 und schließlich vom X-Y-Schreiber 11 aufgezeichnet. Die Logarithmierung erlaubt eine übersichtliche Darstellung des Konzentrationsverlaufs über mehrere Dekaden. In der Auswertezentrale können auch weitere ergänzende Meßwertverarbeitungen, z. B. die Bildung eines 8-Stunden-Mittelwertes, vorgenommen werden. Alternativ kann der 8-Stunden-Mittelwert auch bereits in dem Dosiswarngerät (2-8) gebildet werden.

Die praktische Handhabung des Systems geht in folgender Weise vor sich. Zu Beginn des Arbeitstages wird zunächst die Funktion des Sensors 1 mit einem Testgasgenerator überprüft. Anschließend wird der Sensor mit dem Dosiswarngerät (Komponenten 2 bis 8) kombiniert und während der ganzen Arbeitszeit am Körper der betreffenden Person getragen. Bei Überschreitung von vorgegebenen Grenzwerten bei der Momentanwertkonzentration und bei der Mittelwertkonzentration werden Alarmsignale ausgelöst. Am Ende des Arbeitstages wird das Dosiswarngerät (2 bis 8) vom Gasdetektor 1 entkoppelt und in die Auswertezentrale 9, 10, 11 gesteckt. Hier werden dann wie beschrieben die gespeicherten Meßwerte ausgelesen und nachträglich ein individuelles Protokoll über die Gasbelastung der betreffenden Person während eines Arbeitstages erstellt. Es ist ersichtlich, daß dieses System leicht zu handhaben ist, eine hohe Sicherheit gegenüber Manipulationen bietet und aufgrund der durch die Meßwertverdichtung bedingten Datenreduktion eine überschaubare Protokollierung liefert. So sind bei dem Dosiswarngerät in der Praxis keinerlei Verstellmöglichkeiten von außen her vorgesehen. Die Geräteparameter τ und n · τ sowie die Grenzwerte für die Alarmauslösung werden vom Werk aus fest eingestellt und können vom Träger des Geräts nicht manipuliert werden. Die Gerätekombination Gasdetektor/Dosiswarngerät/Auswertezentrale wird z. B. in der chemischen Industrie zur Expositionsüberwachung von Personen benutzt, die während ihrer Arbeitszeit mit gesundheitsschädlichen Arbeitsstoffen umgehen.

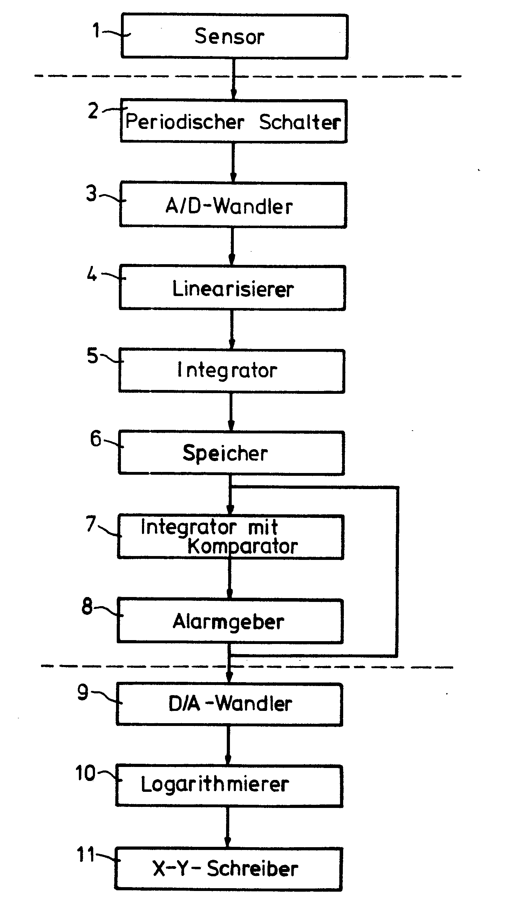

## Ansprüche

1. Vorrichtung zur individuellen Überwachung der Exposition einer Person gegenüber toxischen Gasen mit einem Gassensor (1), der die Gaskonzentration in ein elektrisches Signal umwandelt, einer Einrichtung (11) zur Aufzeichnung des vom Sensor erfaßten Konzentrationsverlaufs und einer Schaltung (5-7) zur Bildung von Konzentrationsmittelwerten, sowie einem bei der Überschreitung eines kritischen Mittelwertes ansprechenden Alarmgeber (8), gekennzeichnet durch

a) einen Schalter (2), der das vom Sensor (1) kommende elektrische Signal periodisch alle 1 bis 10 Sekunden, vorzugsweise 1 bis 2 Sekunden, abfragt, und einen mit dem Sensor (1) verbundenen weiteren Alarmgeber,

b) einen A/D-Wandler (3), der den abgefragten Signalwert digitalisiert,

c) eine erste Integrierstufe (5), die die digitalisierten Meßwerte für eine Mittelwertbildung über einen vorgegebenen Zeitabschnitt τ integriert, wobei τ größer ist als die Zeitkonstante des Sensors und 50 bis 150 Sekunden beträgt,

d) einen Speicher (6), der die durch die Integrierstufe (5) gemittelten Meßwerte speichert,

e) eine zweite Integrierstufe (in 7), die aus den jeweils letzten n Mittelwerten den fließenden Mittelwert über einen Zeitabschnitt n · τ bildet, wobei $2 \leqslant n \leqslant 40$ ist,

f) einen Komparator (in 7), der den fließenden Mittelwert mit einem vorgegebenen Grenzwert vergleicht und mit dem Alarmgeber (8) verbunden ist.

2. Vorrichtung nach Anspruch 1, dadurch gekennzeichnet, daß n in Abhängigkeit von der Toxizität des zu messenden Gases gewählt ist.

3. Vorrichtung nach Anspruch 1 oder 2, dadurch gekennzeichnet, daß bei Sensoren mit nicht-linearer Charakteristik eine Korrekturschaltung (4) zur Linearisierung der digitalisierten Meßwerte vorgesehen ist.

4. Vorrichtung nach Anspruch 3, dadurch gekennzeichnet, daß der periodische Schalter (2), A/D-Wandler (3), die Korrekturschaltung (4), die Integratoren (5 und 7) sowie Speicher (6) und Alarmgeber (8) als Zusatzgerät zum Gassensor (1) in einer Baueinheit zusammengefaßt sind.

5. Vorrichtung nach einem der Ansprüche 1 bis 4, dadurch gekennzeichnet, daß der Ausgang des Speichers (6) an eine Auswertezentrale anschließbar ist, die aus einem D/A-Wandler (9), einer Logarithmierschaltung (10) und einer Meßwertausgabe (11) besteht.

## Claims

1. An apparatus for monitoring the exposure of an individual person to toxic gases, having a gas sensor (1) which converts the gas concentration into an electric signal, a device (11) for recording the concentration gradient detected by the sensor and a circuit (5-7) for forming concentration mean values, and an alarm emitter (8) which sounds when a critical mean value is exceeded, characterised by

a) a switch (2) which periodically, every 1 to 10 seconds, and preferably every 1 to 2 seconds, scans the signal coming from the sensor (1), and a further alarm emitter connected to the sensor (1),

b) an A/D converter (3) which digitalises the scanned signal value,

c) a first integrating stage (5) which integrates the digitalised measured values for forming a mean value over a predetermined time interval τ, τ being greater than the time constant of the sensor and being 50 to 150 seconds,

d) a memory (6) which stores the measured values averaged by the integrating stage (5),

e) a second integrating stage (in 7) which forms the fluid mean value over a time interval n · τ from the last respective n-mean values, wherein 2 ⩽ n ⩽ 40,

f) a comparator (in 7) which compares the fluid mean value with a predetermined critical value and is connected to the alarm emitter (8).

2. An apparatus according to Claim 1, characterised in that n is selected as a function of the toxicity of the gas to be measured.

3. An apparatus according to Claim 1 or 2, characterised in that a correcting circuit (4) is provided for linearising the digitalised measured values in the case of sensors having a nonlinear characteristic.

4. An apparatus according to Claim 3, characterised in that the periodic switch (2), A/D-converter (3), the correcting circuit (4), the integrators (5 and 7) and the memory (6) and alarm emitter (8) are combined in one structural unit as a supplementary device for the gas sensor (1).

5. Process according to one of Claims 1 to 4, characterised in that the output of the memory (6) can be connected to an evaluating centre which consists of a D/A converter (9), a logarithm circuit (10) and a measured value output (11).

**Revendications**

1. Appareil pour la surveillance individuelle de l'exposition d'une personne à des gaz toxiques comportant un capteur de gaz (1) qui transforme la concentration de gaz en un signal électrique, un dispositif (11) pour l'enregistrement de l'évolution de la concentration décelée par le capteur et un circuit (5-7) pour la formation de valeurs moyennes de concentration, ainsi qu'un dispositif d'alarme (8) correspondant au dépassement d'une valeur moyenne critique, caractérisé par

a) un commutateur (2) qui prélève périodiquement toutes les 1 à 10 s, de préférence 1 à 2 s, le signal électrique provenant du capteur (1) et un autre dispositif d'alarme relié au capteur (1),

b) un convertisseur analogique/numérique (3) qui convertit en valeurs numériques les signaux prélevés,

c) un premier étage d'intégration (5) qui intègre sur un intervalle de temps τ prédéterminé les valeurs de mesure converties en valeurs numériques pour une formation de valeur moyenne, τ étant supérieur à la constante de temps du capteur et compris entre 50 et 150 s,

d) une mémoire (6) qui stocke les valeurs de mesure fournies par l'étage d'intégration (5),

e) un second étage d'intégration (en 7), qui forme chaque fois, sur un intervalle de temps n · τ, la valeur moyenne courante à partir des n dernières valeurs moyennes, où 2 ⩽ n ⩽ 40,

f) un comparateur (en 7) qui compare la valeur moyenne courante avec une valeur limite prédéterminée et auquel est relié un dispositif d'alarme.

2. Appareil selon la revendication 1, caractérisé en ce que n est choisi en fonction de la toxicité du gaz mesuré.

3. Appareil selon la revendication 1 ou 2, caractérisé en ce que, dans le cas de capteurs ayant une courbe caractéristique non linéaire, un circuit de correction (4) est prévu pour la linéarisation des valeurs de mesure transformées en valeurs numériques.

4. Appareil selon la revendication 3, caractérisé en ce que le commutateur périodique (2), le convertisseur analogique-numérique (3), le circuit de correction (4), les intégrateurs (5 et 7) ainsi que la mémoire (6) et le dispositif d'alarme (8) sont rassemblés dans une unité de construction comme appareil complémentaire au capteur de gaz (1).

5. Appareil selon l'une des revendications 1 à 4, caractérisé en ce que la sortie de la mémoire (6) peut être reliée à une centrale d'exploitation, qui consiste en un convertisseur numérique-analogique (9), un circuit convertisseur logarithmique (10) et une sortie de valeurs de mesure (11).